# EUROPEAN PATENT APPLICATION

(11) **EP 4 163 361 A1**
(43) Date of publication of application: **12.04.2023**
(21) Application number: 20939603.5
(22) Date of filing: 09.06.2020
(51) Int. Cl.: C12M 1/38

(54) **SINGLE CELL PROCESSING INSTRUMENT**

(71) Applicant: Suzhou Singleron Biotechnologies, Ltd., Suzhou, Jiangsu 215000 (CN); Singleron (Nanjing) Biotechnologies, Ltd., Nanjing, Jiangsu 211800 (CN)
(72) Inventor: ZHOU, Jing, Suzhou, Jiangsu 215000 (CN); ZHANG, Sifu, Suzhou, Jiangsu 215000 (CN); QIU, Yunyan, Suzhou, Jiangsu 215000 (CN); XU, Chuanlai, Suzhou, Jiangsu 215000 (CN)
(74) Representative: Plougmann Vingtoft a/s
(86) International application number: PCT/CN2020/095005
(87) International publication number: WO 2021/248291

(57) **Abstract**

The present disclosure relates to a single cell analysis system. Disclosed herein is an instrument for single cell processing. The instrument comprises: a motor component; a processing component, which comprises a processing chamber inside and multiple first connecting holes; a container, which comprises a sample collecting reservoir, a waste collecting reservoir, multiple sample loading reservoirs, multiple first microchannels, and a second microchannel; a chip, which is connected under the container and forms a gap with the container, the chip comprises a third microchannel, the bottom of the third microchannel comprises a microwell array; a snap component comprises a feeding beam and a snap body, and a first end of the feeding beam connects to the snap body and a second end of the feeding beam connects to the motor component; and a pneumatic component which connects with the multiple first connecting holes.

## Description

### TECHNICAL FIELD

The present application relates to the field of single cell analysis technique, for example, an instrument for high throughput single cell processing.

### BACKGROUND

In the related art, the deoxyribonucleic acid (DNA) or ribonucleic acid (RNA) extraction from a single cell is mainly operated in a semi-automatic way, which is complicated, taking longer operation time with poorer accuracy of the experiment results. Moreover, it is often difficult to process thousands of single cells in parallel.

### SUMMARY

This application discloses a single cell processing instrument. The instrument can separate single cells, label each single cell, and extract DNA or RNA from each single cell. The instrument is easy to operate and provides high-accuracy experimental results.

Disclosed herein is a single cell processing instrument. The instrument comprises a motor component, a processing component, a container, a chip, a snap component, and a pneumatic component. The processing component comprises a processing chamber and multiple first connecting holes. The container locates inside the processing chamber and comprises a sample collecting reservoir, a waste collecting reservoir, multiple sample loading reservoirs, multiple first microchannels, and a second microchannel. A first end of each sample loading reservoir connects with one first connecting hole. A second end of each sample loading reservoir connects with one first microchannel. A first end of the sample collecting reservoir and a first end of the waste collecting reservoir each connects with one first connecting hole. A second end of the sample collecting reservoir and a second end of the waste collecting reservoir both connect with the second microchannel. The chip locates under the container. A snap gap is formed between the chip and the middle portion of the container. The chip comprises a third microchannel. The bottom of the third microchannel comprises a microwell array. The third microchannel comprises an inlet and an outlet. The inlet of the third microchannel connects with the first microchannels. A gap is formed between the inlet of the third microchannel and the container. The outlet of the third microchannel connects with the sample collecting reservoir and the waste collecting revoir through the second microchannel. The snap component comprises a feeding beam and a snap body. A first end of the feeding beam connects with the snap body. A second end of the feeding beam connects with the motor component. The feeding beam is configured to be driven by the motor component to insert the snap body into the snap gap to lift magnetic beads with samples from the bottom of the third microchannel. The pneumatic component connects with multiple first connecting holes and is configured to control air flow to each first connecting hole.

### BRIEF DESCRIPTION OF DRAWINGS

FIG. 1 is a schematic of a single cell processing instrument with the processing component closed.
FIG. 2 is a schematic of a single cell processing instrument with the processing component open.
FIG. 3 is a perspective view of the container from the top.
FIG. 4 is a perspective view of the container from the bottom.
FIG. 5 is a perspective view of a chip.
FIG. 6 is a perspective view of a sealing gasket.
FIG. 7 is a perspective view of an upper cover of the processing component from the top.
FIG. 8 is a perspective view of an upper cover of the processing component from the bottom.
FIG. 9 is a perspective view of the snap component.
FIG. 10 is a top view of an integrated air flow control board with inner structures illustrated.
FIG. 11 is a perspective view of an integrated air flow control board.

### Reference list

- 1: motor component
- 20: first connecting hole
- 2: processing component
- 21: upper cover
- 210: mounting blind hole
- 22: processing component body
- 220: lock notch
- 221: insulation wall
- 222: heating stage
- 2220: sample holder
- 23: second magnet
- 31: container
- 3101: sample collecting reservoir
- 3102: waste collecting reservoir
- 3103: sample loading reservoir
- 3104: first microchannel
- 3105: second microchannel
- 32: first boss
- 320: exhaust hole
- 33: third boss
- 330: mounting hole
- 34: leg
- 41: chip
- 410: third microchannel
- 4101: inlet
- 4102: outlet
- 42: second boss
- 420: inlet reservoir
- 43: fourth boss
- 430: second through hole
- 5: snap component
- 51: feeding beam
- 52: snap body
- 61: air pump
- 62: solenoid valve
- 63: solenoid-valve control board
- 64: integrated air flow control board
- 641: air inlet hole
- 642: air flow channel
- 643: air flow control valve
- 71: sealing gasket
- 710: second connecting hole
- 72: sealing boss
- 73: tenon
- 8: pressure sensor

### DETAILED DESCRIPTION

In the description of the present application, it is to be noted that spatially related or position related terms, including "center", "upper", "lower", "left", "right", "vertical", "horizontal", "in", and "out" are described from the perspective of the drawings, intended only to facilitate the description of the present application and simplify the description, instead of indicating or implying that the instrument or elements must be at a particular area or position or configured and operated at a particular area or position, and thus cannot be construed as a limitation to the present application. Additionally, terms such as "first" and "second" are used for the ease of description and are not to be construed as indicating or implying relative importance. Terms "first position" and "second position" refer to two different positions.

In the description of the present application, it is to be noted that terms such as "mounted", "joined", and "connected" are to be understood in a broad sense unless otherwise expressly specified and limited. For example, the term "connected" may refer to "securely connected" or "detachably connected"; may refer to "mechanically connected" or "electrically connected"; or may refer to "connected directly", "connected indirectly through an intermediary", or "connected inside two components". For those of ordinary skill in the art, the preceding terms can be construed depending on the actual situation.

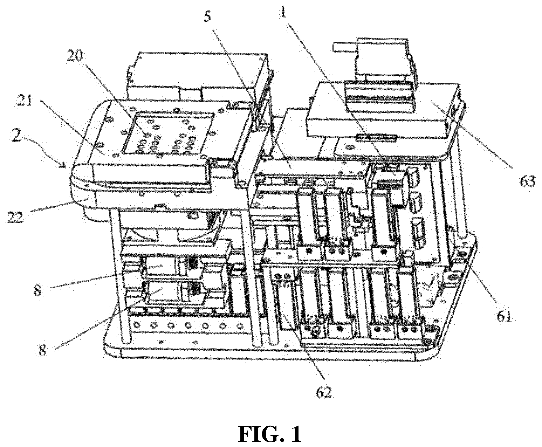

This embodiment discloses a single cell processing instrument. As shown in FIGS. 1 to 8, the instrument comprises a motor component 1, a processing component 2, a container 31, a chip 41, a snap component 5, and a pneumatic component. The processing component 2 comprises a processing chamber inside which the container 31 is located. In one embodiment, two processing chambers locate inside the processing component, and the two processing chambers connect to each other; and two containers 31 locate inside the two processing chambers with one container 31 locates in one processing chamber. When extracting DNAs or RNAs from cells, the single cell processing instrument can have two samples running in parallel so that the experiment speed is greatly improved. In other embodiments, the number of processing chambers may also be one, three, or others, depending on the actual conditions. The processing component 2 comprises twenty first connecting holes 20. The twenty first connecting holes 20 are equally divided into two first connecting hole groups, each having ten first connecting holes 20.

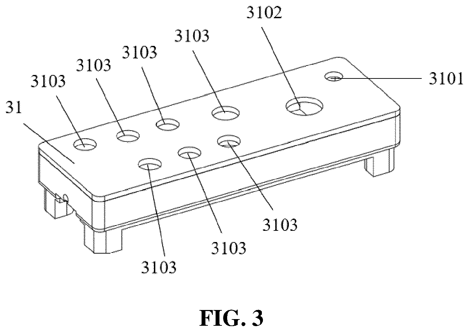

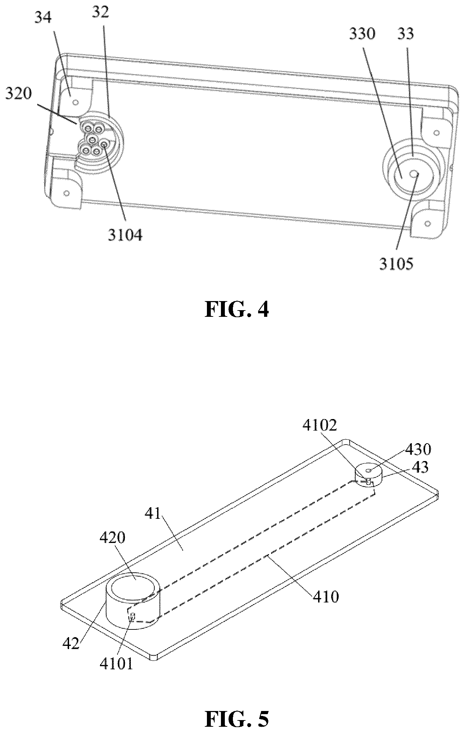

As shown in FIGS. 3 and 4, the container 31 comprises a sample collecting reservoir 3101, a waste collecting reservoir 3102, seven sample loading reservoirs 3103, seven first microchannels 3104, and a second microchannel 3105. A first end of each sample loading reservoir 3103 connects with one first connecting hole 20. A second end of each sample loading reservoir 3103 connects with one first microchannel 3104. A first end of the sample collecting reservoir 3101 and a first end of the waste collecting reservoir 3102 each connects with one first connecting hole 20. A second end of the sample collecting reservoir 3101 and a second end of the waste collecting reservoir 3102 both connect with the second microchannel 3105. As shown in FIG. 5, the chip 41 locates under the container 31. A gap is formed between the chip 41 and the container 31. The chip 41 comprises a third microchannel 410. A snap gap is formed between the chip 41 and the middle portion of the container 31. The bottom of the third microchannel 410 comprises a microwell array. After thousands of suspended single cells are loaded into the third microchannel 410, some cells precipitate into the microwell array after a certain period to isolate from each other. Cells that do not precipitate inside the microwell array are removed in a subsequent washing step. An inlet 4101 of the third microchannel 410 connects with the first microchannels 3104 through the inlet reservoir 420. A gap is formed between the inlet reservoir 420 and the container 31. An outlet 4102 of the third microchannel 410 connects with the sample collecting reservoir 3101 and the waste collecting reservoir 3102 through the second microchannel 3105 and the fourth boss 43.

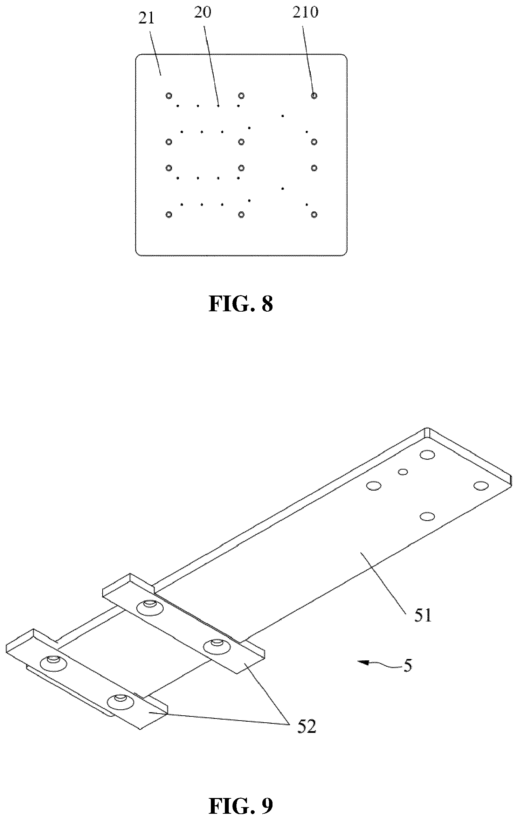

As shown in FIG. 9, the snap component 5 comprises a feeding beam 51 and a snap body 52. A first end of the feeding beam 51 connects to the snap body 52. A second end of the feeding beam 51 connects to the motor component 1. The feeding beam 51 is configured to be driven by the motor component 1 to insert the snap body 52 into the snap gap to lift the magnetic beads with samples from the bottom of the third microchannel 410. In this manner, the samples are suspended in the third microchannel 410. The motor component 1 in this embodiment is a stepper motor.

In one embodiment, the pneumatic component connects with nine first connecting holes 20 of each first connecting hole group. The remaining one first connecting hole 20 in each first connecting hole group is left unconnected as a backup. Thus the processing component 2 is compatible to a container 31 having eight sample loading reservoirs 3103. The pneumatic component can control the on and off, and the pressure of the air flow to each first connecting hole 20.

In other embodiments, the number of the first connecting holes 20, the number of the sample loading reservoirs 3103, and the number of the first microchannels 3104 on the processing component 2 are not limited to the number described in this embodiment and may be other numbers, depending on the actual conditions.

The single cell processing instrument can automatically process thousands of single cells in parallel, label each cell, and then extract DNAs or RNAs from the single cells. An operator loads a cell suspension, various reagents, and a magnetic bead suspension with molecular tags, into different sample loading reservoirs 3103. The pneumatic component controls the on and off, and the pressure of the air flow to each first connecting hole 20 so that the cell suspension, the magnetic bead suspension, a first reagent, a second reagent, a third reagent, and the like flow into the third microchannel 410 of the chip 41 sequentially to react with the cells and extract the desirable samples from the cells to bind to the magnetic beads. The desirable sample is the DNAs or RNAs of the single cells. Finally, with the assistance of the snap component 5, the magnetic beads with extracted samples are lifted from the bottom of the third microchannel 410 so that the magnetic beads with extracted samples are suspended at the upper portion of the third microchannel 410. The magnetic beads with extracted samples are pushed to the outlet 4102 of the third microchannel 410 by the fluid flow and then are collected to the sample collecting reservoir 3101 through the second microchannel 3105. The solvents and the solutions after each reaction in the previous steps are collected into the waste collecting reservoir 3102. After sample collection, the experiment ends. The experimental process is automated, and the accuracy and the repeatability of the experimental results are higher than manual operation. Moreover, since the container 31 and the chip 41 are disposable, it reduces the cross-contamination between different samples. It also reduces the risk of cross-contamination from different samples introduced from the cleaning process of pipes inside an instrument.

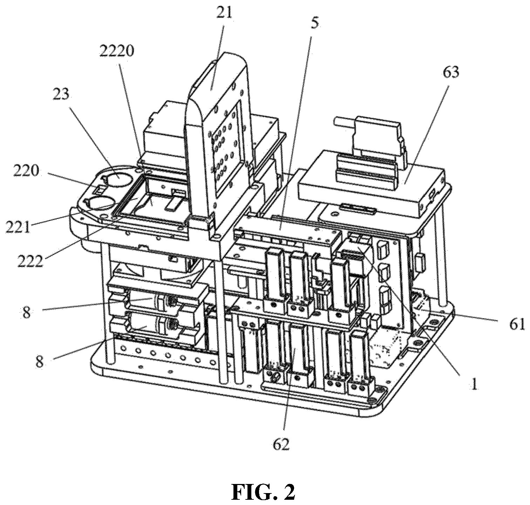

As shown in FIGS. 1 and 2, the pneumatic component comprises an air pump 61, nine solenoid valves 62, and a solenoid-valve control board 63. The solenoid-valve control board 63 is electrically connected to the nine solenoid valves 62. Each solenoid valve 62 relates to one first connecting hole 20 which connects with the container 31. The solenoid-valve control board 63 controls the on and off of the solenoid valves 62 to make the air pump 61 connect with or disconnect from the first connecting holes 20. When the solenoid valves 62 are turned on, the solenoid valves 62 are open so that the air pump 61 connects with the first connecting holes 20 and so that the air pump 61 can push air into the container 31 via positive pressure or suck air or liquid from the container 31 via negative pressure. When the solenoid valves 62 are turned off, the solenoid valves 62 are closed so that the air pump 61 is disconnected from the first connecting holes 20 and so that the air pump 61 cannot push air into the container 31 via positive pressure or suck air or liquid from the container 31 via negative pressure. In one embodiment, the air pump 61 can push air into the container 31 through the first connecting holes 20 to increase the pressure inside the reservoir, and can suck air from the container 31 through the first connecting holes 20 to reduce the pressure inside the reservoir, and can automatically and accurately control the pushing and sucking air volume to precisely adjust the pressure inside the sample loading reservoirs 3103, the sample collecting reservoir 3101, and/or the waste collecting reservoir 3102 in the container 31, thereby increasing the accuracy of the experiment.

In other embodiments, the number of the solenoid valves 62 is not limited to nine as shown in this embodiment and may be other numbers. Moreover, the number of solenoid valves 62 is not less than the total number of the sample collecting reservoir 3101, the waste collecting reservoir 3102, and the sample loading reservoirs 3103.

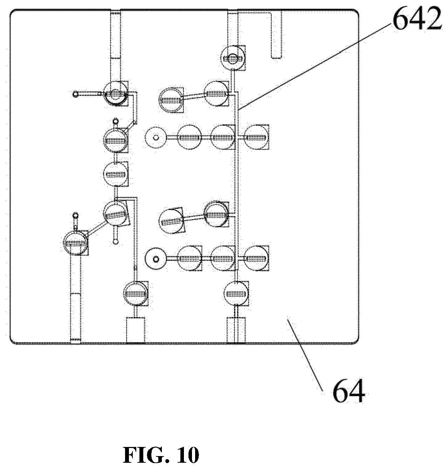

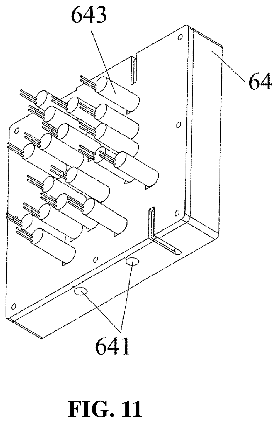

In one embodiment, the pneumatic component comprises multiple connecting tubes (not shown). A first end of the connecting tube connects with the air pump 61. A second end of the connecting tube connects with the first connecting hole 20. Each connecting tube relates to one first connecting hole 20. A solenoid valve 62 is installed on the connecting tube. The solenoid valve 62 controls the connection of the air pump 61 to the connecting tube. In other embodiments, the pneumatic component comprises an integrated air flow control board 64 and a control circuit board. As shown in FIGS. 10 and 11, the integrated air flow control board 64 comprises air inlet holes 641, multiple air flow channels 642, and multiple air flow control valves 643. The air flow control valves 643 electrically connect to the control circuit board. The air inlet holes 641 connect with the air pump 61. A first end of the air flow channel 642 connects with the air inlet hole 641. A second end of the air flow channel 642 connects with the first connecting hole 20. An air flow control valve 643 integrates with one air flow channel 642 to control the continuity of the air flow in the air flow channel 642. Each air flow channel 642 relates to one first connecting hole 20. Each air flow control valve 643 relates to one first connecting holes 20. The control circuit board can individually control the on or off of each air flow control valve 643 to make the air pump 61 connect with or disconnect from the first connecting hole 20.

In one embodiment, the container 31 comprises four legs 34 to facilitate the container 31 positioning in the processing chamber, as shown in FIG. 4. The four legs 34 locate at the four corners of the container 31 to support the container 31. As shown in FIG. 4, a first end of the bottom of the container 31 comprises a first boss 32, and the first boss 32 comprises an exhaust hole 320. The exhaust hole 320 connects with the first microchannels 3104. As shown in FIG. 5, the chip 41 comprises a second boss 42 corresponding to the first boss 32. The second boss 42 comprises an inlet reservoir 420 and a first through hole. The third microchannel 410 connects with the inlet reservoirs 420 through the first through hole. An exhaust gap is formed between the first boss 32 and the second boss 42. In one embodiment, the second boss 42 is mounted inside the first boss 32 by a wall of the second boss 42 enclosed by a wall of the first boss 32 so that the reagents flowing out of the first microchannels 3104 can enter the third microchannel 410 through the inlet reservoir 420. Since the first microchannel 3104 connects with the inlet reservoir 420, as the reagent in the first microchannel 3104 flows out, the air pressure in the first microchannel 3104 is higher than the pressure in the processing chamber outside the container 31. In this manner, any air bubbles existed in the reagent can be released from the exhaust gap between the first boss 32 and the second boss 42, that is, no bubbles exist in the reagent entering the third microchannel 410.

In one embodiment, as shown in FIG. 4, a second end of the bottom of the container 31 comprises a third boss 33. The third boss 33 comprises a mounting hole 330. The mounting hole 330 connects with the second microchannel 3105. As shown in FIG. 5, a fourth boss 43 locates on the chip 41 and corresponds to the third boss 33. The fourth boss comprises a second through hole 430. The second through hole 430 connects with the third microchannel 410. The fourth boss 43 is under the third boss 33. The third boss 33 connects to the fourth boss 43 airtightly. In one embodiment, a sealing ring locates between the third boss 33 and the fourth boss 43, ensuring that the second microchannel 3105 airtightly connects with the third microchannel 410, so that the waste and the sample from the third microchannel 410 can be collected to the waste collecting reservoir and the sample collecting reservoir respectively.

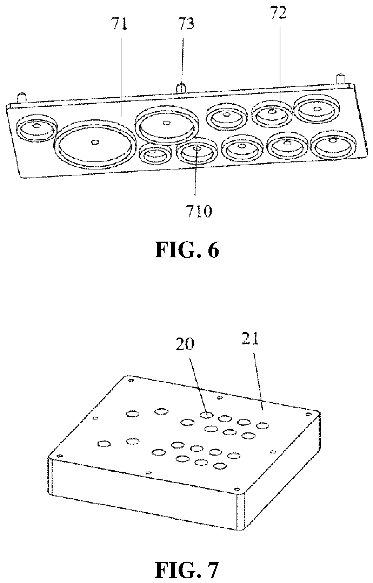

The disclosed single cell processing instrument also comprises a sealing gasket 71, as shown in Fig. 6, to ensure an airtight sealing between the first connecting holes 20 and the container 31. The sealing gasket 71 is a removable rubber sealing gasket. The sealing gasket 71 comprises nine second connecting holes 710. Each second connecting hole 710 relates to one first connecting hole 20. A first end of the second connecting hole 710 connects with one of the first connecting holes 20. A second end of the second connecting hole 710 connects with one of the sample loading reservoirs 3103, or the waste collecting reservoir 3102, or the sample collecting reservoir 3101. A sealing boss 72 locates on the periphery of each second connecting hole 710. The sealing boss 72 ensures an airtight sealing of the sealing gasket 71 with the sample loading reservoirs 3103, the waste collecting reservoir 3102, and the sample collecting reservoir 3101. In one embodiment, as shown in FIG. 6, the sealing gasket 71 comprises six tenons 73. As shown in FIG. 8, the processing component 2 comprises twelve mounting blind holes 210. The twelve mounting blind holes 210 are equally divided into two mounting blind hole groups. The mounting blind holes 210 in each mounting blind hole group locate around the periphery of the first connecting holes 20 in one first connecting hole group. The tenons 73 mount with the mounting blind holes 210 with an interference fit to fix the sealing gasket 71 to the processing component 2. Each one of the six tenons 73 of the sealing gasket 71 relates to one of the six mounting blind holes 210 in one mounting blind hole group. In other embodiments, the number of second connecting holes 710 is not limited to nine and may be other numbers, depending on the total number of sample collecting reservoir 3101, waste collecting reservoir 3102, and sample loading reservoirs 3103. For example, as shown in the figures, the number of second connecting holes 710 is eleven, and the number of sealing bosses 72 is eleven; thus the corresponding total number of sample collecting reservoir 3101, waste collecting reservoir 3102, and sample loading reservoirs 3103 should be eleven.

In one embodiment, the sealing gasket 71 might be detachably mounted to the container 31 by the tenons in an interference fit. In one embodiment, the sealing gasket 71 might be fixed onto the processing component 2. In one embodiment, the sealing gasket 71 might be fixed onto the container 31. In other embodiments, the sealing gasket 71 might be sandwiched between the processing component 2 and the container 31 to provide an airtight sealing between the processing component 2 and the container 31. In other embodiments, the sealing boss 72 might locate on a side of the container 31 facing the sealing gasket 71, or on a side of the sealing gasket 71 facing the container 31, or on two sides of the sealing gasket 71 so that the container 31 is airtightly sealed to the processing component 2.

In one embodiment, as shown in FIG. 1, the processing component 2 comprises an upper cover 21 and a processing component body 22. The upper cover 21 comprises the first connecting holes 20. A first end of the upper cover 21 connects with a first end of the processing component body 22 with rotatable connection. A second end of the upper cover 21 is tightly sealed with the processing component body 22 so that the processing chamber is formed by the upper cover and the processing component body 22. In one embodiment, the upper cover 21 and the processing component body 22 are magnetically connected with a snap fit. In one embodiment, as shown in FIG. 2, the upper cover 21 comprises a protrusion (not shown). The processing component body 22 comprises a lock notch 220 corresponding to the protrusion. The upper cover 21 comprises a first magnet. The processing component body 22 comprises a second magnet 23 corresponding to the first magnet. When the upper cover 21 is snap fitted to the processing component body 22 through the protrusion and the lock notch, the first magnet attracts the second magnet 23 so that the upper cover 21 is tightly sealed with the processing component body 22. When the upper cover 21 is snap fitted to the processing component body 22 and the first magnet and the second magnet 23 are both electrically charged, the electromagnetic attraction force between the upper cover 21 and the processing component body 22 increases, resulting in the sealing of the upper cover 21 and the processing component body 22. In this manner, it reduces the risk of opening the upper cover accidently during the operation of the single cell processing instrument, which introduces a failure of the experiment.

In one embodiment, the processing component body 22 comprises a protrusion, and the upper cover 21 comprises a lock notch 220 corresponding to the protrusion. In one embodiment, the upper cover 21 might be snap fit to but not magnetically connect to the processing component body 22. In one embodiment, the upper cover 21 might be magnetically connect to but not snap fit to the processing component body 22. In other embodiments, the upper cover 21 might locate above the processing component body 22, and the upper cover 21 might slide relatively to the processing component body 22 so that the upper cover 21 can tightly seal to the processing component body 22 to form a processing chamber. That is, when the processing component 2 is closing, the upper cover 21 moves toward the processing component body 22 to seal to the processing component body 22; and when the processing component 2 is opening, the upper cover 21 moves away from the processing component body 22 to detach from the processing component body 22.

As shown in FIG. 2, the processing component body 22 further comprises an insulation wall 221, a heating stage 222, and a cooling fan (not shown). The insulation wall 221 locates around the periphery of the heating stage 222. The upper cover 21, the insulation wall 221, and the heating stage 222 form the processing chamber. The cooling fan locates under the heating stage 222 to cool the heating stage 222. In one embodiment, the heating stage 222 comprises a sample stage, a heating pad, and a temperature sensor. The sample stage stacks on the heating pad. The temperature sensor is configured to measure the temperature of the heating pad. A sample holder 2220 locates on the top of the sample stage. The sample holder 2220 holds the chip 41 and the container 31 with the chip 41 fitting inside the sample holder 2220. There are two sample holders 2220 on one sample stage. Each sample holder 2220 holds one chip 41. The insulation wall 221 locates around the periphery of the heating stage 222 to form a sealed insulation chamber for the heating stage 222. Heating and cooling reagents in the third microchannels 410 of the chip 41 are required during the experimental operation. The heating stage 222 and the insulation wall 221 enables rapid heating and temperature insulation of the samples inside the insulation chamber. The cooling fan enables the rapid temperature adjustment of the heating stage 222, the chip 41, and the samples inside the insulation chamber.

The operation progress inside the processing component 2 is monitored in real time by recording the pressure at an inlet and an outlet of the air pump 61. As shown in FIGS. 1 and 2, the single cell processing instrument further comprises two pressure sensors 8. The two pressure sensors 8 locate under the cooling fan. One pressure sensor is used to measure the air pressure at the inlet of the air pump 61. The other pressure sensor is used to measure the air pressure at the outlet of the air pump 61.

The single cell processing instrument further comprises a controller. The controller electrically connects to the motor component 1, the solenoid-valve control board 63, the air pump 61, the temperature sensor, the heating stage 222, the cooling fan, and the two pressure sensors 8. In one embodiment, the controller might be in a centralized control model or in a distributed control model. For example, the controller might be one independent microcontroller or multiple distributed microcontrollers. The microcontroller controls the motor component 1, the solenoid-valve control board 63, the air pump 61, the temperature sensor, the heating stage 222, the cooling fan, and the two pressure sensors 8.

One exemplary of using the single cell processing instrument to extract RNAs from single cells is described in the following:
S10: The chip 41 and the container 31 are placed into the processing chamber.
S20: The first reagent, the cell suspension, the magnetic bead suspension, the second reagent, and the third reagent are loaded into five sample loading reservoirs 3103. Two sample loading reservoirs 3103 are left empty.
S30: The upper cover 21 is closed by snap fitting to the processing component body 22, simultaneously the first magnet and the second magnet 23 are electrically charged to generate a magnetic attraction force to enforce the closure of the upper cover 21.
S40: The air pump 61 is activated, and the solenoid valve 62 for controlling the connection and disconnection of the air flow to the sample loading reservoir 3103 holding the first reagent is turned on. The air flows into the sample loading reservoir 3101 holding the first reagent through the first connecting hole 20 and the second connecting hole 710. The first reagent is pushed into the inlet reservoir 420 of the chip 41. Then the corresponding solenoid valve 62 is turned off so that the first reagent stops flowing.
S50: The air pump 61 is activated to suck the air from the waste collecting reservoir 3102, thereby dragging the liquid in the third microchannel 410 and sucking the first reagent from the inlet reservoir into the third microchannel 410 to clean the third microchannel 410.
S60: The air pump 61 is activated, and the solenoid valve 62 for controlling the connection and disconnection of the air flow to the sample loading reservoir 3103 holding the cell suspension is turned on. The air flows into the sample loading reservoir 3101 holding the cell suspension through the first connecting hole 20 and the second connecting hole 710. The cell suspension is pushed into the inlet reservoir 420 of the chip 41. Then the corresponding solenoid valve 62 is turned off so that the cell suspension stops flowing.
S70: The air pump 61 is activated to suck the air from the waste collecting reservoir 3102, thereby dragging the liquid in the third microchannel 410 and sucking the cell suspension into the third microchannel 410.
S80: The cells are given a period of time to precipitate into the microwell array 410 at the bottom of the third microchannel 410 of the chip 41 by gravity.
S90: The air pump 61 is activated, and the solenoid valve 62 for controlling the connection and disconnection of the air flow to the sample loading reservoir 3103 holding the first reagent is turned on. The air flows into the sample loading reservoir 3103 holding the first reagent through the first connecting hole 20 and the second connecting hole 710. The first reagent is pushed into the inlet reservoir 420 of the chip 41. Then the corresponding solenoid valve 62 is turned off so that the first reagent stops flowing.
S100: The air pump 61 is activated to suck the air from the waste collecting reservoir 3102, thereby dragging the liquid in the third microchannel 410 and sucking the first reagent into the third microchannel 410 to flush away extra cells not precipitating into the microwells, leaving only a proper amount of single cells in the chip 41.
S110: The air pump 61 is activated, and the solenoid valve 62 for controlling the connection and disconnection of the air flow to the sample loading reservoir 3103 holding the magnetic bead suspension is turned on. The air flows into the sample loading reservoir 3103 holding the magnetic bead suspension through the first connecting hole 20 and the second connecting hole 710. The magnetic bead suspension is pushed into the inlet reservoir 420 of the chip 41. Then the corresponding solenoid valve 62 is turned off so that the magnetic bead suspension stops flowing.
S 120: The air pump 61 is activated to suck the air from the waste collecting reservoir 3102, thereby dragging the liquid in the third microchannel 410 and sucking the magnetic bead suspension into the third microchannel 410.
S130: The air pump 61 is activated, and the solenoid valve 62 for controlling the connection and disconnection of the air flow to the sample loading reservoir 3103 holding the first reagent is turned on. The air flows into the sample loading reservoir 3103 holding the first reagent through the first connecting hole 20 and the second connecting hole 710. The first reagent is pushed into the inlet reservoir 420 of the chip 41. Then the corresponding solenoid valve 62 is turned off so that the first reagent stops flowing.
S140: The air pump 61 is activated to suck the air from the waste collecting reservoir 3102, thereby dragging the liquid in the third microchannel 410 and sucking the first reagent into the third microchannel 410 to flush away extra magnetic beads, leaving only a proper amount of magnetic beads in the chip 41.
S150: The air pump 61 is activated, and the solenoid valve 62 for controlling the connection and disconnection of the air flow to the sample loading reservoir 3103 holding the second reagent is turned on. The air flows into the sample loading reservoir 3103 holding the second reagent through the first connecting hole 20 and the second connecting hole 710. The second reagent is pushed into the inlet reservoir 420 of the chip 41. Then the corresponding solenoid valve 62 is turned off so that the second reagent stops flowing.
S160: The air pump 61 is activated to suck the air from the waste collecting reservoir 3102, thereby dragging the liquid in the third microchannel 410 and sucking the second reagent into the third microchannel 410 to perform a biochemical reaction so that the RNAs of cells are released from the single cells, and bound with molecular structures on the surface of the magnetic beads to form magnetic beads with RNAs.
S170: The motor component 1 is activated to drive the snap body of the snap component 5 to enter the snap gap. The magnetic force generated by the snap body lifts the magnetic beads with RNAs from the bottom of the third microchannel 410 to make the magnetic beads with RNAs suspended in the third microchannel 410, and then the motor component 1 retrieves the snap body of the snap component 5 back to its original position.
S180: The air pump 61 is activated, and the solenoid valve 62 for controlling the connection and disconnection of the air flow to the sample loading reservoir 3103 holding the third reagent is turned on. The air flows into the sample loading reservoir 3103 holding the third reagent through the first connecting hole 20 and the second connecting hole 710. The third reagent is pushed into the inlet reservoir 420 of the chip 41. Then the corresponding solenoid valve 62 is turned off so that the third reagent stops flowing.
S190: The air pump 61 is activated to suck the air from the sample collecting reservoir, thereby dragging the liquid in the third microchannel 410 and sucking the third reagent into the third microchannel 410 to collect the suspended magnetic beads with RNAs into the sample collecting reservoir 3101.
S200: Turn off the electromagnets associated with the upper cover 21 and the processing component body 22 to open the processing component 2; the magnetic beads with RNAs are retrieved from the sample collecting reservoir 3101; and the container 31 and the chip 41 are discarded. At this point, the experiment is completed.

It is to be noted that in each step, the chip 41 and the processing chamber 2 are heated or cooled by the temperature control system according to the temperature requirements of different reagents and the temperature requirements of the biochemical reactions. The process of heating the reagent in the chip 41 by the heating stage 222 and the process of cooling the reagent in the chip 41 by the cooling fan are not described in the above process. In actual operation, the appropriate temperature required for each step may be obtained by programming the controller. At the same time, the temperature sensor measures the temperature of the heating pad in real time to monitor the temperature of the reagents. During the operation of the single cell processing instrument, the cooling fan is always on to dissipate the heat from the chip 41 and the processing chamber.

This embodiment shows only one exemplary of the operation of the single cell processing instrument. Other exemplary of the operation of the instrument is not excluded.

Compared with the related art, the single cell processing instrument disclosed here is fully automatic, using less time in the whole experimental process, reducing the failure rate to extract the DNAs or RNAs from thousands of single cells in parallel compared to a manual operation. Therefore, it increases the success rate of the experiments, and improves the accuracy of the experimental results. Moreover, the sealing gasket, the container, and the chip of the single cell processing instrument are disposable so that it reduces the risk of cross-contamination from different samples and the inadequate cleaning of the instrument pipes.

## Claims

1. A single cell processing instrument, comprising:
a motor component (1);
a processing component (2) comprising a processing chamber and a plurality of first connecting holes (20);
a container (31) located inside the processing component and comprising a sample collecting reservoir (3101), a waste collecting reservoir (3102), a plurality of sample loading reservoirs (3103), a plurality of first microchannels (3104) and a second microchannel (3105), wherein a first end of each of the plurality of sample loading reservoirs (3103) connects with one of the plurality of first connecting holes (20), a second end of each of the plurality of sample loading reservoirs (3103) connects with one of the plurality of first microchannels (3104), a first end of the sample collecting reservoir (3101) and a first end of the waste collecting reservoir (3102) each connects with one first connecting hole (20) of the plurality of first connecting holes (20), and a second end of the sample collecting reservoir (3101) and a second end of the waste collecting reservoir (3102) both connect with the second microchannel (3105);
a chip (41) located under the container (31), wherein a snap gap is formed between the chip (41) and a middle portion of the container (31), the chip (41) comprises a third microchannel (410), the third microchannel (410) comprises an inlet (4101) and an outlet (4102), the inlet (4101) connects with multiple first microchannels (3104) of the plurality of first microchannels (3104), a snap gap is formed between the inlet (4101) and the container (31), the outlet (4102) connects with the sample collecting reservoir (3101) and the waste collecting reservoir (3102) separately through the second microchannel (3105), and the third microchannel (410) comprises a microwell array at a bottom of the third microchannel (410);
a snap component (5) comprising a feeding beam (51) and a snap body (52), wherein a first end of the feeding beam (51) connects to the snap body (52), a second end of the feeding beam (51) connects to the motor component (1), and the feeding beam (51) is configured to be driven by the motor component (1) to insert the snap body (52) into the snap gap to lift a sample comprising magnetic beads located inside the third microchannel (410); and
a pneumatic component connecting with multiple first connecting holes (20) of the plurality of first connecting holes (20) and configured to control air flow to each of the multiple first connecting holes (20) of the plurality of first connecting holes.

2. The single cell processing instrument according to claim 1, wherein the pneumatic component comprises an air pump (61), a plurality of solenoid valves (62), and a plurality of connecting tubes, wherein a solenoid valve (62) of the plurality of solenoid valves (62) locates on a connecting tube of the plurality of connecting tubes, a first end of the connecting tube connects with the air pump (61), a second end of the connecting tube connects with one first connecting hole (20) of the plurality of first connecting hole (20), each of the plurality of connecting tubes relates to one of the plurality of first connecting holes (20), each of the plurality of solenoid valves (62) relates to one of the plurality of first connecting holes (20), and the solenoid valve (62) is configured to make the air pump (61) connect with the connecting tubes or disconnect from the connecting tubes.

3. The single cell processing instrument according to claim 1, wherein the pneumatic component comprises an air pump (61) and an integrated air flow control board (64), wherein the integrated air flow control board (64) comprises air inlet holes (641), a plurality of air flow channels (642), and a plurality of air flow control valves (643), wherein the air inlet holes (641) connect with the air pump (61), a first end of an air flow channel (642) of the plurality of air flow channels (642) connects with the air inlet hole (641), a second end of the air flow channel (642) connects with one first connecting hole (20) of the plurality of first connecting holes (20), an air flow control valve (643) of the plurality of air flow control valves (643) is configured to be integrated in the air flow channels (642) and controls air flow in the air flow channel (642), each of the plurality of air flow channels (642) relates to one of the plurality of first connecting holes (20), each of the plurality of air flow control valves (643) relates to one of the plurality of air flow channels (642), and the air flow control valve (643) is configured to make the air pump (61) connect with the first connecting holes (20) or make the air pump (61) disconnect from the first connecting holes (20).

4. The single cell processing instrument according to claim 1, further comprising a sealing gasket (71) sandwiched between the processing component (2) and the container (31) so that the processing component (2) is air tightly connected to the container (31), wherein the sealing gasket (71) comprises a plurality of second connecting holes (710), a first end of the second connecting hole (710) of the plurality of second connecting holes (710) connects with one first connecting hole (20) of the plurality of first connecting holes (20), a second end of the second connecting hole (710) connects with one sample loading reservoir (3103) of the plurality of sample loading reservoirs (3103), or the waste collecting reservoir (3102), or the sample collecting reservoir (3101).

5. The single cell processing instrument according to claim 1, wherein the container (31) comprises a first boss (32), the first boss (32) comprises an exhaust hole (320), the exhaust hole (320) connects with each of the plurality of first microchannels (3104), the chip (41) comprises a second boss (42), the second boss (42) comprises a inlet reservoir (420), the inlet reservoir (420) connects with the third microchannel (410), the first boss (32) locates inside the second boss (42), or the second boss (42) locates inside the first boss (32), and an exhaust gap is formed between the first boss (32) and the second boss (42).

6. The single cell processing instrument according to claim 1, wherein the container (31) further comprises a third boss (33), the third boss (33) comprise a mounting hole (330), the mounting hole (330) connects with the sample collecting reservoir (3101) and the waste collecting reservoir (3102) separately through the second microchannel (3105), the chip (41) comprises a fourth boss (43), the fourth boss (43) comprises a through hole (430), the through hole (430) connects with the third microchannel (410), the fourth boss (43) is under the third boss (33), and the third boss (33) is air tightly connected to the fourth boss (43).

7. The single cell processing instrument according to claim 1, wherein the processing component comprises an upper cover (21) and a processing component body (22), wherein the upper cover (21) comprises the plurality of first connecting holes (20), a first end of the upper cover (21) is connected with one end of the processing component body (22) with a rotatable connection, and a second end of the upper cover (21) is tightly sealed to the processing component body (22) so that the processing chamber is formed between the upper cover (21) and the processing component body (22).

8. The single cell processing instrument according to any one of claims 1 to 6, wherein the processing component (2) comprises an upper cover (21) and a processing component body (22), wherein the upper cover (21) locates above the processing component body (22), the upper cover (21) comprises the plurality of first connecting holes (20), the upper cover (21) is slidably disposed on the processing component body (22) so that the upper cover (21) is tightly sealed to the processing component body (22) so that the processing chamber is formed between the upper cover (21) and the processing component body (22).

9. The single cell processing instrument according to claim 7 or claim 8, wherein the upper cover (21) and the processing component body (22) are configured in at least one of the following manners:
the upper cover (21) and the processing body (22) snap fit to each other; or
the upper cover (21) and the work box body (22) are magnetically connected to each other.

10. The single cell processing instrument according to claim 7 or claim 8, wherein the processing component body (22) comprises an insulation wall (221) and a heating stage (222), wherein the insulation wall (221) locates around a periphery of the heating stage (222); and the upper cover (21), the insulation wall (221) and the heating stage (222) define the processing chamber.

11. The single cell processing instrument according to any one of claims 1 to 8, further comprising at least two pressure sensors (8), wherein at least one of the at least two pressure sensors (8) is configured to measure pressure of an inlet of an air pump (61) of the pneumatic component, and at least one of the at least two pressure sensors (8) is configured to measure pressure of an outlet of the air pump (61) of the pneumatic component.
